# EUROPEAN PATENT APPLICATION

(11) **EP 4 484 565 A1**
(43) Date of publication of application: **01.01.2025**
(21) Application number: 22934613.5
(22) Date of filing: 18.07.2022
(51) Int. Cl.: C12P 13/02, C12P 41/00, C12N 9/78, C12N 15/55, C12N 15/70, C12N 1/21, C12N 11/089, C12N 11/14, C07C 253/30

(54) **METHOD FOR ENZYMATIC SYNTHESIS OF BRIVARACETAM CHIRAL INTERMEDIATE**

(30) Priority: 02.04.2022 CN 202210350130
(71) Applicant: Zhejiang University of Technology, Hangzhou, Zhejiang 310014 (CN)
(72) Inventor: ZHENG, Renchao, Hangzhou, Zhejiang 310014 (CN); ZHENG, Yuguo, Hangzhou, Zhejiang 310014 (CN); ZHAN, Kan, Hangzhou, Zhejiang 310014 (CN); WU, Zheming, Hangzhou, Zhejiang 310014 (CN)
(74) Representative: karo IP
(86) International application number: PCT/CN2022/106271
(87) International publication number: WO 2023/184791

(57) **Abstract**

Disclosed is a method for enzymatic synthesis of a brivaracetam chiral intermediate, i.e., a method for synthesizing a brivaracetam chiral intermediate (R)-3-cyanohexanoic acid by catalyzing the hydrolysis of 3-cyanohexanitile using an enzyme with nitile hydrolysis activity, and the enzyme with nitrile hydrolysis activity is obtained by carrying out a single mutation or a double mutation on an amino acid at position 140 or an amino acid at position 175 in an amino acid sequence as set forth in SEQ ID NO.2. Compared with a wild type, the nitrilase mutant of the present invention has the activity increased by 10 times, an ee value increased to 300 or more from 39, a substrate conversion rate of 45%, and a product ee which can reach 98.5%, and the yield of (R)-3-aminomethyl-hexanoic acid by catalytic hydrogenation synthesis using (R)-3-cyanohexanoic acid reaches 85% or more. The present invention features a short synthesis route, mild reaction conditions, and high atom economy, and can be applied to the industrial synthesis of the brivaracetam intermediate.

## Description

### (1)TECHNICAL FIELD OF THE INVENTION

The present invention relates to a method for enzymatic synthesis of a brivaracetam chiral intermediate, and more particularly to a method for synthesizing a brivaracetam chiral intermediate using an enzyme having nitrilase activity.

### (2) Background Art

Brivaracetam, whose chemical name is (2S)-2-[(4R)-2-oxo-4-propyl-1-pyrrolidinyl] butyramide, is a new generation of antiepileptic drug developed by UCB at Belgium, and its chemical structure is shown in Formula (I):

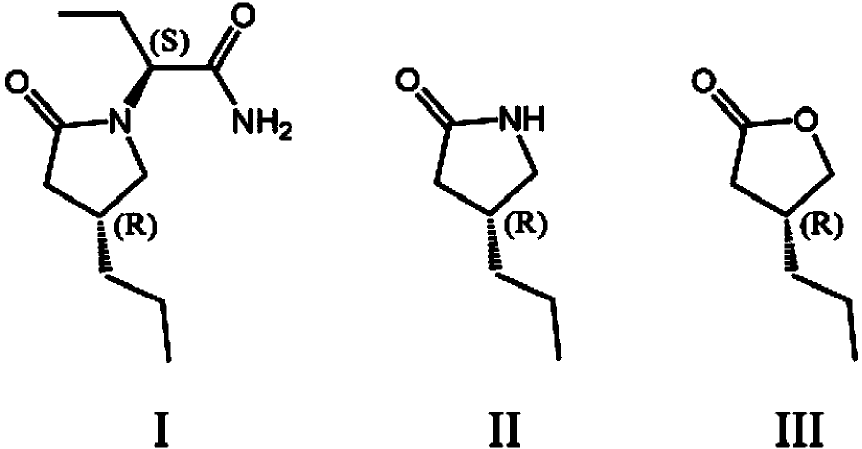

Brivaracetam is clinically used to treat systemic epilepsy, is a high affinity ligand for synaptic vesicle protein 2A (SV2A), while having certain inhibitory property on sodium ion channels. In February 2016, FDA approved brivaracetam to be marketed as a new generation of drugs for the treatment of epilepsy and has a broad market prospect. Compound (II) and compound (III) are key chiral intermediates of numerous brivaracetam synthetic routes, which can be synthesized from (R)-3-aminomethyl hexanoic acid (IV). Therefore, the construction of a green and efficient, intrinsically safe and highly economical (R) -3-carbamomethylhexanoic acid (IV) preparation method is of great significance for the industrial production of HIV.

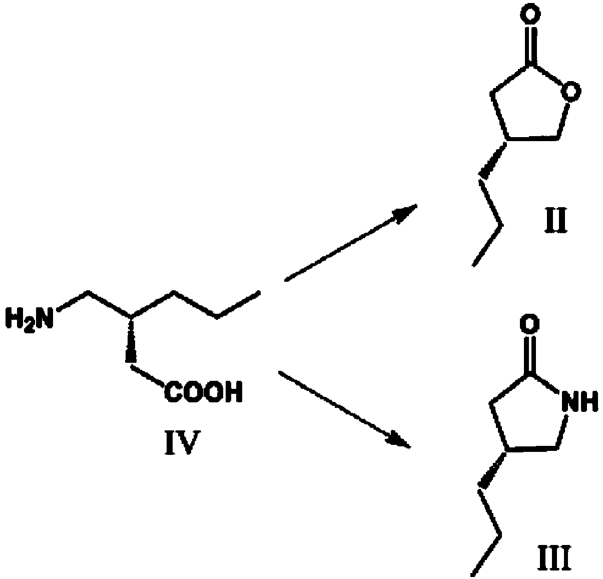

At present, the synthetic routes of compound (IV) mainly have the following two:

Route 1: compound (VIII) is subjected to resolution with chiral phenylethylamine to obtain compound (VII), and then compound (VII) is subjected to Huffman degradation under the action of sodium hypochlorite or sodium hypobromate to obtain compound (IV) (CN113045468A). This method has complex chiral resolution step, low resolution yield, and produces a small amount of isomerization impurities, which is not conducive to industrial production.

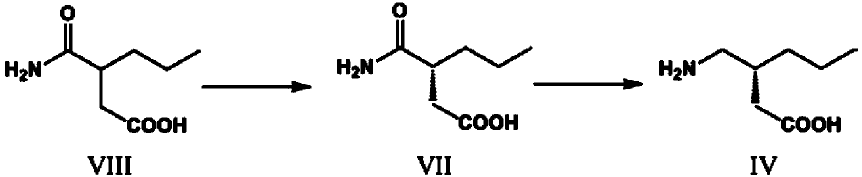

Route II: compound (X) is used as a starting material to react with diisobutylamine and bromoacetate to synthesize compound (IX), and then compound (IX) is subjected to catalytic reduction amination by transaminase to prepare chiral compound (IV) (WO2016075082). The synthesis process of the method is complex, the ee value of the product is only about 90%, and it is difficult to meet the requirements of industrial production.

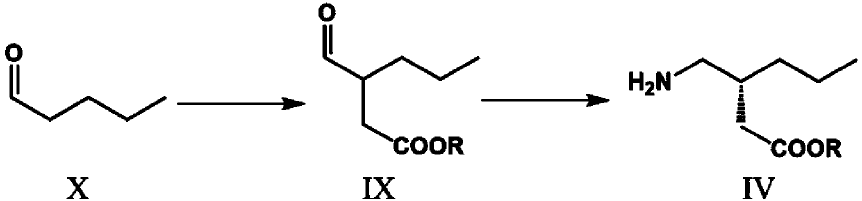

Biocatalyzed organic nitrile hydrolysis has significant advantages such as strict selectivity, mild reaction conditions, sustainable development, and the like, and has become an important method for preparing chiral carboxylic acid. The present invention involves efficiently synthesizing (R)-3-cyanohexanoic acid (V) by nitrilase region and stereoselective hydrolysis of 3-cyanohexanoic acid (V), and then subjecting (R)-3-cyanohexanoic acid (V) to hydrogenation reduction to synthesize (R)-3-aminomethyl hexanoic acid (IV), and then using (R)-3-aminomethyl hexanoic acid (IV) to prepare the brivaracetam intermediate compound (II) and compound (III).

### (3) Summary of the Invention

The purpose of the present invention is to provide a method for enzymatic synthesis of a brivaracetam chiral intermediate, in particular, a method for synthesizing (R)-3-cyanohexanoic acid by using an enzyme having nitrilase activity to catalyze hydrolysis of 3-cyanohexanenitrile, and then performing hydrogenation reduction on (R)-3-cyanohexanoic acid to synthesize (R)-3-aminomethyl hexanoic acid, so as to construct a brand-new enzymatic synthesis process of brivaracetam, which is of great significance for realizing industrial green synthesis of brivaracetam.

The technical solution adopted by the present invention is as follows:

In a first aspect, the present invention provides a method for enzymatic synthesis of a brivaracetam chiral intermediate, wherein the method is to synthesize (R)-3-cyanohexanoic acid by using an enzyme having nitrilase activity to catalyze the hydrolysis of 3-cyanohexanenitrile, and specifically comprises: forming a reaction system by using wet cells obtained by inducing expression of a recombinant genetically engineered strain containing the gene encoding the enzyme having nitrilase activity as a catalyst, 3-cyanohexanenitrile (IV) as a substrate, and a buffer solution of pH 7-8 or water as a medium, carrying out the reaction at 20-40°C and 200-600 rpm (preferably 30° C, 300 rpm), after the reaction is completed, subjecting the reaction solution to separation and purification to obtain (R)-3-cyanohexanoic acid (V); wherein the enzyme having nitrilase activity is nitrilase, and the nitrilase is obtained by performing single mutation or double mutation on the amino acid at position 140 or position 175 of the amino acid sequence shown in SEQ ID NO. 2.

Preferably, the nitrilase is obtained by performing one of the following mutations on amino acid sequence shown in SEQ ID NO 2: (1) mutating the tryptophan at position 140 to glycine (W140, the nucleotide sequence is as shown in SEQ ID NO. 3, and the amino acid sequence is as shown in SEQ ID NO. 4); (2) mutating the tryptophan at position 140 to glycine, and the methionine at position 175 to threonine (W140G/M175T, the nucleotide sequence is as shown in SEQ ID NO. 5, and the amino acid sequence is as shown in SEQ ID NO. 6).

Preferably, in the reaction system, the amount of the catalyst is 1-10 g/L (preferably 3 g/L) based on the dry weight of the wet cells, and the concentration of the substrate is 100-300 g/L (preferably 150 g/L).

Preferably, the catalyst is prepared as follows: inoculating the recombinant genetically engineered strain containing the gene encoding the enzyme having nitrilase activity into an LB liquid medium containing 50 µg/mL kanamycin, culturing it overnight at 37° C, inoculating it at an inoculation volume of 2%(v/v) into an LB medium containing 50 µg/mL kanamycin, culturing it at 37°C and 150 rpm until the concentration of the cells reach OD₆₀₀=0.6, adding IPTG with a final concentration of 0.1 mM, performing induction culture at 28°C for 12 h, , then subjecting the resulting culture to centrifugation at 4°C at 12000 rpm for 10 min, and collecting wet cells.

Preferably, the catalyst is a crude enzyme by disrupting and extracting the wet cells, a pure enzyme after purification of the crude enzyme, immobilized wet cells or immobilized enzyme; that is, the catalyst may be whole cells of the recombinant genetically engineered strain expressing the nitrilase, or may be used in the form of an unpurified crude enzyme, or may be used in the form of partially purified or completely purified enzyme. The nitrilase mutant of the present invention can also be made into a biocatalyst in the form of immobilized enzyme or immobilized cells using immobilization techniques known in the art.

Preferably, the 3-cyanohexanenitrile is prepared as follows: subjecting a mixture of n-butyraldehyde, piperidine, ethyl cyanoacetate and n-hexane to the reaction at 30-40°C for 10-15 h (preferably 35° C, 12 h), and distilling the resulting mixture under reduced pressure to remove the solvent to obtain an oil; adding isopropanol and an aqueous solution containing potassium cyanide to the oil, maintaining the reaction at 30-40°C for 3-8 h (preferably 35°C for 5 h), and carry out a reflux reaction at 80-1 10°C for 3-8 h (preferably 95°C for 5 h); cooling the resulting reactant to room temperature, diluting it with water, subjecting the mixture to extraction with methyl tert-butyl ester, washing the extract with water for three times, drying it with anhydrous magnesium sulfate, and concentrating it until no liquid flows out under a vacuum condition to obtain an oily product 3-cyanohexanenitrile (VI); wherein the mass ratio of piperidine to n-butyraldehyde, ethyl cyanoacetate and potassium cyanide is 1: 80-90: 100-150: 60-100 (preferably 1: 85-86: 122-123: 70); and the ratio of the volume of n-hexane to the total amount of n-butyraldehyde, piperidine, and ethyl cyanoacetate is ranging from 1 to 5 mL/g (preferably 2 to 3 mL/g); the volume ratio of the isopropanol to the n-hexane is 5: 3; the volume ratio of the aqueous solution containing the potassium cyanide to the n-hexane is 5: 3; the volume ratio of the water for dilution to the n-hexane is 2: 3; and the volume ratio of the methyl tert-butyl ester to the n-hexane is 5: 3.

The method for preparing (R)-3-aminomethyl hexanoic acid (IV) from (R)-3-cyanohexanoic acid (V) comprises: taking (R)-3-cyanohexanoic acid into a hydrogenation reactor, adding a catalyst Raney nickel, introducing nitrogen to replace air for 3 times, then introducing hydrogen to a final pressure of 2 MPa, and carry out a reaction for 9 h at room temperature, then stopping stirring, and emptying hydrogen; subjecting the reaction solution to filtration, recovering the catalyst in the filter cake, and purifying the filtrate to obtain (R)-3-aminomethyl hexanoic acid; wherein the mass ratio of the (R)-3-cyanohexanoic acid to the catalyst is 1: 1-5 (preferably 1: 1.5). The purification of the filtrate refers to: centrifuging the sampled reaction solution at 8000 rpm for 10 min, then adding ethyl acetate to the supernatant according to a volume ratio of 3: 1 to extract the remaining substrate, adjusting the pH of the resulting aqueous phase to 1 with 6M HCl, adding ethyl acetate for extraction, adding an aqueous NaOH solution with a pH = 12 into the resulting organic phase for back extraction, and the resulting aqueous phase to obtain an (R)-3-aminomethyl hexanoic acid aqueous solution.

In a second aspect, the present invention provides a nitrilase mutant for synthesizing a brivaracetam chiral intermediate (R)-3-cyanohexanoic acid, wherein the mutant is obtained by performing single mutation or double mutation on the amino acid at position 140 or position 175 of the amino acid sequence shown in SEQ ID NO. 2.

The present application further relates to a coding gene of the nitrilase mutant, a recombinant vector comprising the coding gene, and a recombinant genetically engineered strain constructed by the recombinant vector. The recombinant vector is a pET-28a plasmid, and the recombinant genetically engineered strain uses E coli BL21 (DE3) as a host Bactera.

The present invention uses n-butyraldehyde (XI) and ethyl cyanoacetate as raw materials to prepare (R)-3-aminomethyl hexanoic acid (IV), and the synthetic route is as follows:

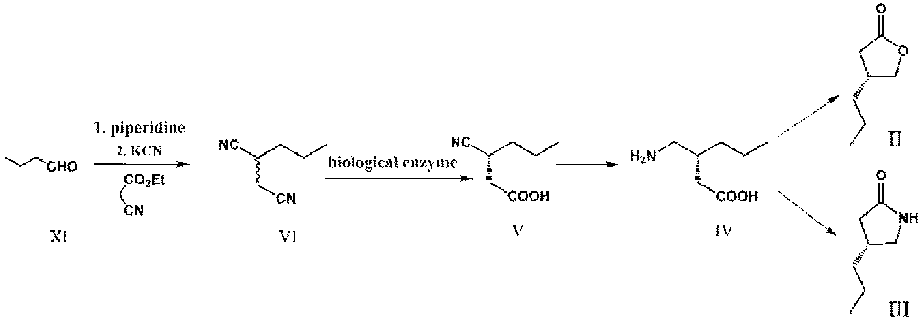

3-cyanohexanenitrile (VI) is prepared by a chemical method with n-butyraldehyde (XI) and ethyl cyanoacetate as raw materials; and (R)-3-cyanohexanoic acid (V) is prepared by biological catalysis with 3-cyanohexanenitrile (VI) as a substrate. (R)-3-aminomethyl hexanoic acid (IV) is obtained by catalytic hydrogenation with (R)-3-cyanohexanoic acid (V) as a substrate. Finally, the compound (II) and (III) are obtained by esterification or amidation with (R)-3-aminomethyl hexanoic acid (IV) as a substrate.

Compared with the prior art, the beneficial effects of the present invention are mainly embodied in that the present invention provides a method for the enzymatic synthesis of brivaracetam chiral intermediates, that is, a method for synthesizing (R)-3-cyanohexanoic acid by catalytic hydrolysis of 3-cyanohexanenitrile (VI) by using nitrilase. The nitrilase is a mutant from Grilamella burkholderia nitrilase. Compared with the wild type, the activity of the mutant is improved by 10 times, and the E value is increased from 39 to 300 or above. The nitrilase mutant is used to catalyze the synthesis of (R)-3-cyanohexanoic acid, the conversion rate of the substrate is 45%, and the ee of the product can reach 98 %; then (R)-3-cyanohexanoic acid is used to synthesize (R)-3-aminomethyl hexanoic acid via catalytic hydrogenation, and the yield of (R)-3-aminomethyl hexanoic acid (IV) iss above 85%. The synthesis route is short, the reaction conditions are mild, the atom economy is high, and the synthesis route can be applied to industrial synthesis of brivaracetam intermediates.

### (4)BRIEF DESCRIPTION OF THE DRAWINGS

FIG. 1 is the ¹H NMR graph of 3-cyanohexanenitrile.
FIG. 2 is an ¹³C NMR plot of 3-cyanohexanenitrile.
FIG. 3 is a gas chromatogram of (R) -3 cyanohexanoic acid synthesized by using a nitrilase to catalyze the hydrolysis of 3-cyanohexanenitrile.

### (5) DETAILED DESCRIPTION OF THE PREFERRED EMBODIMENTS

The present invention is further described below with reference to specific examples, but the scope of protection of the present invention is not limited thereto: the room temperature in the present application is 25-30° C.

### Example 1. Synthesis of 3-Cyanohexanenitrile (VI)

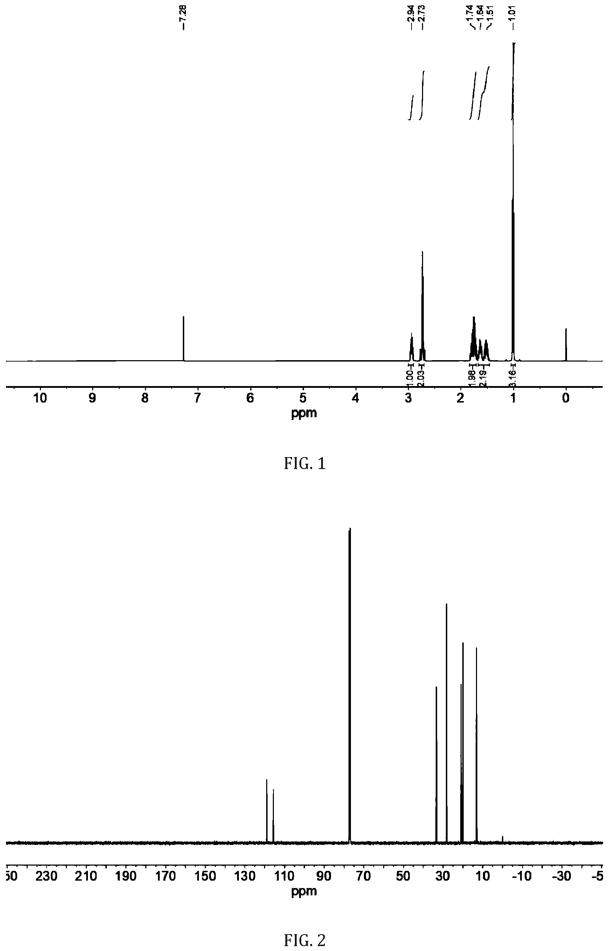

A mixture of n-butyraldehyde (51.4 g), piperidine (0.6 g), ethyl cyanoacetate (73.7g) and n-hexane (300 mL) was continuously reacted at 35°C for 12 h, and the resulting mixture was distilled under reduced pressure to remove the solvent to obtain an oil. 500 mL of isopropanol and 500 mL of an aqueous solution containing 42.0 g of potassium cyanide were added to the oil, the mixture was maintained at 35°C for 5 hours, and refluxed at 95°C for 5 hours. After the mixture was cooled to room temperature, it was diluted with water (200 mM) and extracted with methyl tert-butyl ester (500 mL), the extract was washed three times with water, dried with anhydrous magnesium sulfate and concentrated to no liquid to flow out under vacuum to give 73 g of an oily product 3-cyanohexanenitrile (VI) (CAS: 1116-53-6), with a yield of 92%. ¹H NMR is shown in FIG. 1, and ¹³C NMR is shown in FIG. 2.

¹H NMR(400MHz , CDCl₃) : (δ ^{,} ppm) ^{,} 3.0-2.9(m ^{,} 1H) ^{,} 2.8-2.7(d ^{,} 2H) ^{,} 1.9-1.7(m ^{,} 2H) ^{,} 1.7-1.4(m ^{,} 2H) ^{,} 1.1-0.9(t ^{,} 3H).

¹³C NMR(400MHz ^{,} CDCl₃) : (δ ^{,} ppm) ^{,} 120 ^{,} 116 ^{,} 34 ^{,} 28 ^{,} 22 ^{,} 20 ^{,} 14.

### Example 2: Construction of Nitrilase Mutant

### 1. Construction of Recombinant Nitrilase Pg-Nit

A nitrilase Pg-Nit gene (GenBank NO.WP006050412.1, nucleotide sequence was shown as SEQ ID NO. 1) derived from *Paraburkholderia graminis* and a vector pET-28a were digested with *NCO* I and *Hind* III, respectively, a T4 ligase was used to construct a pET-*Pg*-Nit plasmid; the pET-Pg-Nit plasmid was heat-transformed into *E*. *coli* BL21 (DE3) competent cells, resuscitated and then coated on an LB plate containing 50 µg/ml kanamycin for culture, and cultured overnight at 37° C.

The colonies on the plate were inoculated into an LB liquid medium containing 50 µg/mL kanamycin, cultured overnight at 37° C, inoculated into an LB medium containing 50 µg/mL kanamycin at an inoculation volume of 2%, and cultured at 37° C and 150 rpm until the concentration of the cells reached OD₆₀₀=0.6, IPTG with a final concentration of 0.1 mM was added, induction culture was performed at 28°C for 12h, then the culture was centrifuged at 4°C at 12000 rpm for 10 min, and wild-type wet cells *E. coli* BL21 (DE3)-PG-NIT were collected.

SEQ ID No.1 
ATGGGTAAAGTTGTCAAAGCCGCTGCTGTTCAATTTTCTCCAGTTCTGTACA
GCCGCGAAGCAACCGTAGCAAAAGTCGTACAGAAGATCCACGAACTGGGTCTG
AAAGGCGTGCAATTCGCTACCTTCCCGGAAACCGTTGTCCCGTACTACCCGTATT
TCGCTGCAGTTCAGACTGGTATCGAGCTGCTGAGCGGTTCCGAACACCTGCGTC
TGCTGGAGCAGGCGGTTACTGTTCCTAGCGCGGCTACGGATGCAATCGGTAAAG
CTGCTCGTGAAGCAGGTATGGTTGTATCTATCGGCGTGAACGAGCGCGACGGTG
GCACGCTGTACAACACCCAACTGCTGTTTGATGCCGATGGCACGCTGATTCAGC
GTCGCCGTAAAATCACCCCAACTCACTTCGAACGTATGATTTGGGGTCAGGGTG
ATGGTTCTGGTCTGCGTGCGGTTGATTCCGCCGTGGGCCGCATTGGTCAGCTGG
CGTGTTTCGAACATAACAACCCACTGGCCCGTTACGCGATGATCGCTGATGGCG
AACAAATCCATTCTGCGATGTATCCGGGCAGCGCTTTTGGCGAAGGTTTTGCGC
AGCGTATGGAAATCAACATTCGTCAGCACGCACTGGAATCCGGCGCGTTCGTAG
TCAACGCAACCGCATGGCTGGATGCGGATCAGCAGGCACAAATTATGAAAGACA
CCGGCTGCGGCATTGGTCCAATTAGCGGTGGTTGTTTCACCACCATTGTTTCCCC
GGACGGTATGCTGATGGCTGAACCGCTGCGCTCTGGTGAGGGCGAGGTCATCGT
TGACCTGGACTTTGCACAGATCGATCGTCGTAAAATGCTGATGGACGCTGCCGG
TCATTACAACCGTCCGGAACTGCTGTCTCTGATGATCGATCGTACCCCTACCGCG
CATGTACATGAACGTGCGCCGCACTCCCTGCCGGTAAGCGACAAAGCGGACGA
CGACGTGCGCACCCAAGCGGCTGCAGTCGCGGGTTCCCGCCTCGAGATT.
SEQ ID No.2

MGKVVKAAAVQFSPVLYSREATVAKVVQKIHELGLKGVQFATFPETVVPYY
PYFAAVQTGIELLSGSEHLRLLEQAVTVPSAATDAIGKAAREAGMVVSIGVNERDG
GTLYNTQLLFDADGTLIQRRRKITPTHFERMIWGQGDGSGLRAVDSAVGRIGQLAC
FEHNNPLARYAMIADGEQIHSAMYPGSAFGEGFAQRMEINIRQHALESGAFVVNA
TAWLDADQQAQIMKDTGCGIGPISGGCFTTIVSPDGMLMAEPLRSGEGEVIVDLDF
AQIDRRKMLMDAAGHYNRPELLSLMIDRTPTAHVHERAPHSLPVSDKADDDVRT
QAAAVAGSRLEI.

### 2 Construction of Nitrilase Single Mutant

Site-directed mutagenesis was performed on the Trp at position 140 of the amino acid sequence shown in SEQ ID NO. 2 of the wild-type nitrilase PG-NIT, and the corresponding primers were designed, the primer sequence was shown in Table 1. The pET-Pg-Nit plasmid containing the wild-type nitrilase gene PG-NIT shown in SEQ ID NO. 1 was used as a template, the primers in Table 1 were used to perform full plasmid PCR amplification, and saturated mutation was performed on tryptophan at position 140, that is, the nucleotide corresponding to position 140 was replaced with NNK instead of the original codon.

**TABLE 1: Primer Design Table for Site-Directed Saturated Mutant at position 140**

| Primer name | Primer sequences |
|---|---|
| W140-F | ATGATTNNKGGTCAGGGTGATGGTTCTGG |
| W140-R | TCACCCTGACCMNNAATCATACGTTCGAAGTGAG |
| M175-F | TACGCGNNKATCGCTGATGGCGAACAAAT |
| M175-R | CCATCAGCGATMNNCGCGTAACGGGCCAGTGGGT |

| | |
|---|---|
| Note: N=A/G/C/T,K=G/T,M=A/C. | |

The method for constructing recombinant nitrilase Escherichia coli by PCR amplification and PCR product transformation refers to patent CN202011383565.4.

The mutant wet cells were prepared according to the method in step 1. The composition of the biocatalytic reaction system (10 mL) was: 50 mL KH₂PO₄-K₂HPO₄ buffer (pH 7.2), 5 g/L of 3-cyanohexanenitrile (VI) prepared by the method of Example 1, 0.1 g of wild-type wet cells or mutant wet cells. The reaction solution was preheated at 30°C for 10 min, and reacted at 30°C and 600 rpm for 15 min. After 500 µL of the reaction solution was sampled, 20 µL of 2M HCl was added to terminate the reaction and the mixture was extracted with ethyl acetate, the upper organic phase was dried with anhydrous sodium sulfate, 30 µL of methanol and 15 µL of diazomethane were added for mixing, and the content of the substrate 3-cyanohexanenitrile and product (R)-3-cyanohexanoic acid (V, CAS:528566-19-6) was determined by gas chromatography, the conversion of the substrate, the enantiomeric excess (*ee*) of the product and the enantiomeric selectivity (E value) of the enzyme were calculated.

Definition of Enzyme Activity: The amount of enzyme required to produce 1 µmol of product per minute at 30°C is one unit (U).

The model of gas chromatography was 7890N (Agilent), and the model of the capillary column was BGB -175 (BGB Analytik Switzerland). The chromatographic conditions were as follows: the sample injection amount is 1.0 µL, the temperature of sample inlet and the detector were both 250° C. The column temperature was maintained at 120°C for 17 minutes, then the temperature was raised to 170°C at a program heating rate of 10° C./min and maintained for 8 min. The carrier gas was high-purity helium, the flow rate is 1.0 mL/min, and the shunt ratio is 50: 1.

**TABLE 2. Activity and E value changes before and after the nitrilase mutation**

| nitrilase | activity (U/g) | *E* |
|---|---|---|
| *Pg*-Nit | 27 | 39 |
| W140G | 136 | >300 |
| W140A | 35 | 60 |
| W140V | 17 | 55 |
| W140M | 57 | 76 |
| W140L | 125 | 30 |

The results showed that the activity of the mutant PG-NIT-W140G (the nucleotide sequence was as shown in SEQ ID NO. 3, the amino acid sequence as shown in SEQ ID NO. 4) reached 136 U/g, which was 5 times that of the wild type, and the stereoselectivity was also greatly improved, and the E value was raised from 39 for the wild type to 300 or above 300.

### 3. Construction of Nitrilase Double Mutants

On the basis of the mutant PG-NIT-W140 G with improved activity and stereoselectivity in step 2, the plasmid containing the mutant PG-NIT-W140 G wet cells was extracted, PG-NIT-W140 G plasmid was used as a mutant template, M175 F/R in Table 1 was used as a primer, the whole plasmid PCR amplification was performed by using the method in step 2, and the mutant PG-NIT-W140G/M175T was constructed (the nucleotide sequence was as shown in SEQ ID NO 5, and the amino acid sequence was as shown in SEQ ID NO 6).

The mutant Pg-Nit-W140G/M175T was transformed into E coli BL21 (DE3) according to the method in step 2, and the corresponding wet cells were prepared; the enzyme activity and the E value were calculated by using the method in step 2; as shown in Table 3, the hydrolysis activity of the mutant PG-NIT-W140g/M175T reached 271 U/g, which was twice the mutant PG-NIT-W140g.

**TABLE 3. Activity and Stereoselectivity Comparison of Nitrilases**

| Nitrilase | Amino acid sequence | Activity( U/g) | *E* |
|---|---|---|---|
| *Pg*-Nit | SEQ ID NO.2 | 27 | 39 |
| W140G | SEQ ID NO.4 | 136 | >300 |
| W140G /M175T | SEQ ID NO.6 | 271 | >300 |

### Example 3. Whole Cell Culture of Recombinant Nitrilase

The wild-type E.coli BL21(DE3)-Pg-Nit, recombinant nitrilase mutant E.coli BL21(DE3)-Pg-Nit-W140G/M175T constructed in Example 2 were inoculated into an LB liquid medium containing 50 µg/mL kanamycin respectively, cultured overnight at 37° C, inoculated into an LB medium containing 50 µg/mL kanamycin at an inoculation volume of 2%, and cultured at 37°C and 150 rpm to a cell concentration OD₆₀₀= 0.6, then IPTG was added with a final concentration of 0.1 mM, the culture was cultured at 28°C for 12 h, centrifuged at 4°C and 12000 rpm for 10 min, and wet cells were collected to obtain the wild-type PG-Nit wet cells and mutant W140G/M175T wet cells, respectively.

### Example 4. Synthesis of (R)-3-Aminomethylhexanoic Acid (I) Catalyzed by Nitrilase

### 1. Synthesis of (R)-3-cyanohexanoic Acid from 3-cyanohexanenitrile

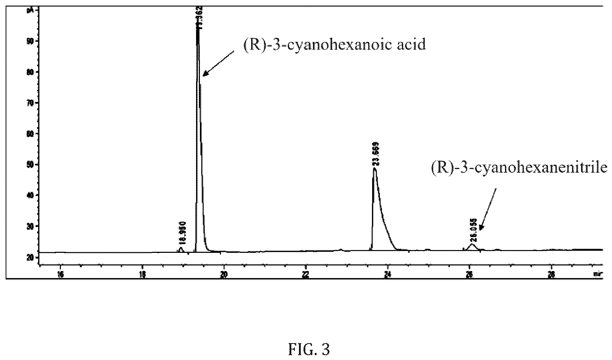

In a 1L reaction system, water was used as a reaction medium, the wet cells of the recombinant nitrilase mutant W140G/M175T and the wild-type PG-Nit obtained in Example 3 were added according to the amount of 3 g/L (dry weight) respectively, then the substrate 3-cyanohexanenitrile prepared by the method of Example 1 with a final concentration of 100 g/L was added, and the reaction was performed at 30°C and 600 rpm for 24h. The reaction solution was sampled, the gas chromatography was used for detecting the concentration of substrate 3-cyanohexanenitrile and product (R) -3-cyanoproproic acid as described in Example 2 (Figure 3). After the sampled reaction solution was centrifuged at 8000 rpm for 10 min, 300 mL of ethyl acetate was added to extract the remaining substrate, the pH of the aqueous phase was adjusted to 1 through 6M HCl, 300 mL of ethyl acetate was added for extraction, 100 mL of aqueous NaOH solution with pH = 12 was added into the organic phase for back extraction, the aqueous phase was taken to obtain (R)-3-cyanohexanoic acid aqueous solution, and water was added to adjust to a concentration of 200 g/L.

The results showed that after 24 hours of reaction, the substrate conversion rate of the wild-type PG-NIT was 35%, and the ee value of the product (R)-3-cyanohexanoic acid was 92.0%; the substrate conversion rate of the mutant W140G/M175T was 45%, and the ee value of the product (R)-3-cyanohexanoic acid is 98.4%.

MS (m/z): C₇H₁₁NO₂ (M + H⁺) theoretical calculated value 141.1, found 141.1.

### 2. Hydrogenation of (R)-3-cyanohexanoic acid to synthesize (R)-3-aminomethyl hexanoic acid

The (R)-3-cyanohexanoic acid aqueous solution obtained in step 1 (containing 20 g of (R)-3-cyanohexanoic acid) was added to a 500 mM hydrogenation reactor, 30 g of catalyst Raney nickel was added, nitrogen was introduced to replace air 3 times, hydrogen was introduced to the final pressure of 2° C, stirring was stopped after reacting at room temperature for 9 h, and hydrogen was vented. After the reaction solution was subjected to suction filtration, the catalyst in the filter cake was recovered, the filtrate was sampled and the content of (R)-3-cyanohexanoic acid residue was detected by the gas phase detection as described in Example 2, and the content of (R)-3-aminomethyl hexanoic acid (CAS: 1314557-10-2) was detected by high performance liquid chromatography.

The liquid phase detection conditions were: a chromatographic column J&KCHEMICAQC-18column; the detection wavelength was 210 nm; the mobile phase was pH 6.3 phosphate buffer (final concentration 3.4 g/L): methanol = 850: 150 (v/v).

The results showed that in the system (R)-3-cyanohexanoic acid had no residue, the conversion rate was 100%, and the yield of (R)-3-aminomethyl hexanoic acid reached 85.2%.

MS (m/z): C₇H₁₁NO₂(M + H⁺) theoretical calculated value 145.2, found 145.2.

### Example 5. Synthesis of (R)-3-Aminomethylhexanoic Acid (II) Catalyzed by Nitrilase

### 1. Synthesis of (R)-3-cyanohexanoic Acid from 3-cyanohexanenitrile

The wet cells in step 1 in Example 4 were changed into recombinant nitrilase mutant W140g/M175T, the substrate addition concentration was changed to 150 g/L, the reaction time was changed to 13 h, and the other operations were the same to obtain 200 g/L (R)-3-cyanohexanoic acid aqueous solution. The results showed that the substrate conversion rate of the reaction 13 h, the mutant W140g/m 175 T was 45.0%, and the ee value of the product (R)-3-cyanohexanoic acid was 98.5%. MS (m/z): C₇ H₁₁ NO₂ (M + H⁺) theoretical calculated value 141.1, found 141.1.

### 2. Hydrogenation of (R)-3-cyanohexanoic acid to synthesize (R)-3-aminomethyl hexanoic acid

100 mM (R)-3-cyanohexanoic acid aqueous solution obtained in step 1 (containing 20 g of (R)-3-cyanohexanoic acid) was added to a 500 mM hydrogenation reactor, 30 g of catalyst Raney nickel was added, nitrogen was introduced to replace air 3 times, hydrogen was introduced was a final pressure of 2MPa, stirring was stopped after reaction at room temperature for 9 h, and hydrogen was vented. The reaction solution was detected using the method of Example 4.

The results showed that in the system (R)-3-cyanohexanoic acid had no residue, the conversion rate was 100%, and the yield of (R)-3-aminomethyl hexanoic acid reached 85.0%.

### Example 6. Synthesis of (R)-3-Aminomethyl Hexanoic Acid (III) Catalyzed by Nitrilase

### 1. Synthesis of (R)-3-cyanohexanoic Acid from 3-cyanohexanenitrile

The wet cells in Example 4 were changed to a recombinant nitrilase mutant W140G/M175 T, the substrate addition concentration was changed to 300 g/L, and the other operations were the same, to obtain a 200 g/L (R)-3-cyanohexanoic acid aqueous solution. The results showed that after reaction for 24 h, the conversion of mutant W140G / M175T was 39.0%, and the product (R) -3-cyanohexanate was 98.9%.

GC-MS (m/z): C₇H₁₁NO₂ (M + H^{+□}) theoretical calculated value 141.1, found 141.1.

### 2. (R)-3-cyanohexanoic acid hydrogenation to synthesize (R)-3-aminomethyl hexanoic acid

The (R)-3-cyanohexanoic acid aqueous solution obtained in step 1 (containing 20 g of (R)-3-cyanohexanoic acid) was added to a 500 mM hydrogenation reactor, 30 g of catalyst Raney nickel was added, nitrogen was introduced to replace air 3 times, hydrogen was introduced with the final pressure of 2MPa, stirring was stopped after reaction at room temperature for 9 h, and hydrogen was vented. The reaction solution was detected using the method of Example 4.

The results showed that in the system (R)-3-cyanohexanoic acid had no residue, the conversion rate was 100%, and the yield of (R)-3-aminomethyl hexanoic acid reached 86.3%.

### Example 7. Synthesis of (R)-3-Aminomethylhexanoic Acid (4) catalyzed by Nitrilase

### 1. Synthesis of (R)-3-cyanohexanoic Acid

The wet cells in step 1 in Example 5 were changed to immobilized cells, and the immobilization method was as follows: first, wet cells were resuspended with 100mM NaHCO₃ aqueous solution to obtain 100g/L bacterial suspension, stirring was performed for 20 min without obvious blocks, the diatomite was added in an amount of 6 g/L and continued to stir for 20 min, the aqueous solution of 25% glutaraldehyde with a volume concentration of 25% was added in an amount of 10 mM/L after stirring for 1 h, stirring was continued for 1 h, then suction filtration was performed, liquid was discarded, the obtained immobilized cells were washed with water, and finally the nitrilase immobilized cells were obtained. The addition amount of immobilized cells was 15 g/L, and the other operations were the same to obtain 200 g/L of (R)-3-cyanohexanoic acid aqueous solution. The results showed that the reaction was 13 h, the substrate conversion was 44.5%, and the ee value of the product (R)-3-cyanohexanoic acid was 98.7%. MS (m/z): C₇ H_{1 1} NO₂ (M + H⁺) theoretical calculated value 141.1, found 141.1.

### 2. (R)-3-cyanohexanoic acid hydrogenation to synthesize (R)-3-aminomethyl hexanoic acid

100 mM (R)-3-cyanohexanoic acid aqueous solution obtained in step 1 (containing 20 g of (R)-3-cyanohexanoic acid) was added to a 500 mM hydrogenation reactor, 30 g of catalyst Raney nickel was added, nitrogen was introduced to replace air 3 times, hydrogen was introduced with a final pressure of 2MPa. Stirring was stopped after reaction at room temperature for 9 h, and hydrogen was vented. The reaction solution was detected using the method of Example 4.

The results show that in the system (R)-3-cyanohexanoic acid has no residue, the conversion rate is 100%, and the yield of (R)-3-aminomethyl hexanoic acid reaches 85.5%.

## Claims

1. A method for enzymatic synthesis of a brivaracetam chiral intermediate, which is **characterized in that** the method is a method for synthesizing a brivaracetam chiral intermediate (R)-3-cyanohexanoic acid by using an enzyme with nitrilase activity to catalyze the hydrolysis of 3-cyanohexanenitrile.

2. The method according to claim 1, wherein the method is as follows: forming a reaction system by using wet cells obtained by inducing expression of a recombinant genetically engineered strain containing the gene encoding an enzyme having nitrilase activity as a catalyst, 3-cyanohexanenitrile as a substrate, and a buffer solution of pH 7-8 or water as a medium, carrying out the reaction at 20-40°C and 200-600 rpm, after the reaction is completed, subjecting the reaction solution to separation and purification to obtain (R)-3-cyanohexanoic acid.

3. The method according to claim 1 or 2, **characterized in that** the enzyme having nitrilase activity is nitrilase.

4. The method according to claim 3, wherein the nitrilase is obtained by performing single mutation or double mutation on the amino acid at position 140 or position 175 of the amino acid sequence shown in SEQ ID NO 2.

5. The method according to claim 4, wherein the amino acid sequence of the nitrilase is shown in SEQ ID NO. 4 or SEQ ID NO. 6.

6. The method according to claim 2, wherein in the reaction system, the amount of the catalyst is 1-10 g/L based on the dry weight of the wet cells, and the concentration of the substrate is 100-300 g/L.

7. The method of claim 2, wherein the catalyst is prepared as follows: inoculating the recombinant genetically engineered strain containing the gene encoding the enzyme having nitrilase activity into an LB liquid medium containing 50 µg/mL kanamycin, culturing it overnight at 37° C, inoculating it into an LB culture medium containing 50 µg/mL kanamycin at a volume concentration of 2%, culturing it at 37°C and 150 rpm until the concentration of the cells reaches OD₆₀₀= 0.6, adding IPTG with a final concentration of 0.1 mM, performing induction culture at 28°C for 12 h, then subjecting the resulting culture to centrifugation at 4°C and 12000 rpm for 10 min, and collecting wet cells.

8. The method according to claim 2, wherein the catalyst is a crude enzyme obtained by disrupting and extracting the wet cells, a pure enzyme after purification of the crude enzyme, immobilized wet cells or immobilized enzyme.

9. A nitrilase mutant for synthesizing a brivaracetam chiral intermediate of claim 1, wherein the mutant is obtained by performing single mutation or double mutation on the amino acid at position 140 or position 175 of the amino acid sequence shown in SEQ ID NO 2.

10. A recombinant genetically engineered strain containing the gene encoding the nitrilase mutant of claim 9.
